(19) Europäisches Patentamt
European Patent Office
Office européen des brevets

(11) **EP 1 698 361 B1**

(12) **EUROPÄISCHE PATENTSCHRIFT**

(45) Veröffentlichungstag und Bekanntmachung des Hinweises auf die Patenterteilung:
**15.10.2008 Patentblatt 2008/42**

(51) Int Cl.:
*A61M 1/16* (2006.01)    *G07C 3/00* (2006.01)

(21) Anmeldenummer: 05101697.0

(22) Anmeldetag: **04.03.2005**

(54) **Dialysemaschine mit Wartungsanzeige**

Dialysis machine comprising indication of maintenance

Machine de dialyse avec moyens pour indiquer la maintenance

(84) Benannte Vertragsstaaten:
**AT BE BG CH CY CZ DE DK EE ES FI FR GB GR HU IE IS IT LI LT LU MC NL PL PT RO SE SI SK TR**

(43) Veröffentlichungstag der Anmeldung:
**06.09.2006 Patentblatt 2006/36**

(73) Patentinhaber: **B. Braun Avitum AG**
**49215 Glandorf (DE)**

(72) Erfinder:
• **Dolgos, Sandor**
**2000 Szentendre (HU)**
• **Schin, G. Robert**
**1029 Budapest (HU)**

• **Moll, Stefan**
**34212 Melsungen (DE)**

(74) Vertreter: **Selting, Günther et al**
**Patentanwälte**
**von Kreisler Selting Werner,**
**Bahnhofsvorplatz 1**
**Deichmannhaus am Dom**
**50667 Köln (DE)**

(56) Entgegenhaltungen:
WO-A-96/40316          DE-A1- 3 828 224
DE-A1- 4 131 247       DE-A1- 10 015 450
DE-A1- 10 148 214      DE-A1- 19 832 825

**Beschreibung**

Die Erfindung betrifft eine Dialysemaschine zur extrakorporalen Blutbehandlung, nämlich zur Reinigung des Blutes von Schadstoffen, und insbesondere eine Dialysemaschine mit Wartungsanzeige.

Dialysemaschinen enthalten ein komplexes System zur Reinigung des Blutes, das dem Körper eines Patienten entnommen wird. Die Dialysemaschine enthält einen Blutkreislauf und einen Dialysatkreislauf, die beide durch unterschiedliche Kammern eines Dialysators verlaufen, in dem die Schadstoffe dem Blut entzogen werden. Das Dialysegerät enthält Flüssigkeitsfördersysteme mit zahlreichen Pumpen zum Fördern des Dialysats und des Blutes sowie zur Herstellung des Dialysats aus verschiedenen Flüssigkeiten und zur Temperierung. Außerdem ist ein komplexes Steuer- und Überwachungssystem mit zahlreichen Sensoren und Aktuatoren vorhanden. Die Sensoren umfassen beispielsweise Leitfähigkeitssensoren, Drucksensoren, Temperaturfühler u. dgl. Die Aktuatoren umfassen hauptsächlich Pumpen und Ventile. Da der Ausfall einer Dialysemaschine für den daran angeschlossenen Patienten lebensbedrohend werden kann, muss jede Maschine nach bestimmten Vorschriften regelmäßig gewartet werden. Solche präventiven Wartungen sind unter Umständen behördlich vorgeschrieben. Außerdem haben die einzelnen Herstellerfirmen eigene Wartungsvorschriften. Die Wartungsvorschriften sehen in der Regel Wartungsintervalle in Form bestimmter festgelegter Zeitspannen vor. Am häufigsten sind eine einjährige oder zweijährige Wartung. Aus wirtschaftlichen Gründen werden jedoch zunehmend längere Wartungsintervalle gefordert. Zu lange Wartungsintervalle bergen allerdings die Gefahr von unvorhergesehenen Geräteausfällen während der Behandlung und erzeugen damit ebenfalls hohe Kosten durch Gerätestillstand und Kundenbeanstandung.

EP 1 331 589 A2 beschreibt ein medizinisches Gerät, das im Stande ist, auftretende Fehler zu erkennen und über ein Fernsystem an das Wartungspersonal zu übertragen, um dieses über eine anstehende Reparatur zu informieren. Hierbei erfolgt die Reparatur erst nach dem Erkennen eines Fehlers, was bei Dialysemaschinen häufig zu spät ist.

In WO 96/40316 ist eine Dialysemaschine mit Wartungsanzeige beschrieben. Bei dieser wird für die einzelnen Komponenten jeweils ein Testwert festgelegt. Der Testwert wird mit einem Schwellenwert verglichen, der die Grenze der normalen Verwendbarkeit der Komponente angibt. Jeder Fall, in dem der Testwert den Schwellenwert übersteigt, wird gezählt, wobei ein Wartungszählwert entsteht. Der Wartungszählwert repräsentiert die Anzahl der Fehlfunktionen der betreffenden Komponente. Bei geringer Maschinenbenutzung können zu lange Wartungsintervalle auftreten.

In DE 198 32 825 A1 ist eine Vorrichtung zum computergestützten Betreiben von Einrichtungen zur Bestrahlung des menschlichen Körpers beschrieben. Diese Einrichtung berücksichtigt hauttypspezifische Parameter der Bestrahlungseinrichtungen. Es erfolgt eine individuelle Erfassung des Hauttyps sowie der Bestrahlungsgewohnheiten des jeweiligen Nutzers. Für jede der Bestrahlungseinrichtungen erfolgt eine Erfassung der Betriebsstunden, wobei anhand der jeweiligen Betriebsstundenzahl für die jeweilige Bestrahlungseinrichtung aus einer vorgegebenen Alterungs- oder Lebensdauerkurve der Bestrahlungslampen oder -röhren ein Bestrahlungskorrekturwert ermittelt wird, welcher als Bestrahlungszeitberechnungskoeffizient dem Steuercomputer zuführbar ist. Hierbei erfolgt eine Berücksichtigung der Alterungseffekte der Maschinenkomponenten bei der Berechnung der Bestrahlungsstärke bzw. der Bestrahlungsdauer.

Der Erfindung liegt die Aufgabe zugrunde, eine Dialysemaschine mit Wartungsanzeige zu schaffen, die auf eine anstehende oder empfohlene Wartung hinweist und dabei zu kurze wie auch zu lange Wartungsintervalle in Abhängigkeit von der Maschinenbenutzung ausschließt.

Die Dialysemaschine mit Wartungsanzeige nach der vorliegenden Erfindung ist durch den Patentanspruch 1 definiert. Sie enthält eine Messvorrichtung für die Messgrößen: Dauer des laufenden Wartungsintervalls (seit der letzten durchgeführten Wartung) und Betriebsstundenzahl innerhalb des laufenden Wartungsintervalls. Aus diesen Messgrößen wird mittels einer Recheneinrichtung die Länge des Wartungsintervalls bestimmt. Die Recheneinrichtung arbeitet nach einem vorgegebenen Algorithmus, der den für die Wartung vorgesehenen Zeitpunkt bestimmt und den Anwender rechtzeitig noch vor einem Geräteausfall oder einer Störung informiert bzw. warnt. Durch die Kombination der beiden genannten Parameter wird verhindert, dass einerseits ein stark benutztes Gerät zu selten gewartet wird und damit ein Sicherheitsrisiko darstellt, und dass andererseits ein selten benutztes Gerät zu oft gewartet wird und dadurch unnötige Mühen und Kosten aufgewandt werden.

Neben den beiden genannten Faktoren können auch andere Faktoren in die Berechnung des Wartungsintervalls eingehen, wie beispielsweise das Alter der Dialysemaschine, die Gesamtzahl der bisher geleisteten Betriebsstunden sowie auch die Zahl der Betriebsstunden von besonders kritischen Komponenten.

Ein einfaches Kriterium sieht vor, dass das Ende des Wartungsintervalls bestimmt wird durch das Erreichen einer Obergrenze für die Betriebsstundenzahl, jedoch nicht früher als eine Untergrenze der Dauer des laufenden Wartungsintervalls. Dies bedeutet, dass in jedem Fall das Wartungsintervall mit einer bestimmten Mindestzeitdauer ausgestattet ist. Erst bei Überschreiten der Mindestzeitdauer gibt die Betriebsstundenzahl die Obergrenze für das Wartungsintervall an. Auch andere Algorithmen können aus den beiden genannten Messgrößen gebildet werden.

Das Lebensalter der Dialysemaschine kann dadurch berücksichtigt werden, dass mit zunehmendem Lebensalter die Obergrenze für die Betriebsstundenzahl erniedrigt und/oder die Untergrenze der Dauer des laufenden Wartungsintervalls ebenfalls erniedrigt wird. Anstelle des zeitlichen Lebensalters der Dialysemaschine kann auch die Gesamtzahl der bisher abgeleisteten Betriebsstunden als Alterungskriterium berücksichtigt werden.

Eine Dialysemaschine enthält bestimmte Maschinenkomponenten, die besonders sicherheitsrelevant sind. Dies sind beispielsweise bestimmte Pumpen oder Ventile. Solche Maschinenkomponenten können im Rahmen der Erfindung einzeln überwacht werden, wobei jede dieser speziellen Maschinenkomponenten ihre eigene Historie hat, die gemessen oder ermittelt und aufgezeichnet wird. Dabei wird die Zahl der Betriebsstunden dieser speziellen Maschinenkomponente unabhängig von dem sonstigen Betrieb der Dialysemaschine gezählt. Dialysemaschinen können mit unterschiedlichen Programmen bzw. in unterschiedlichen Betriebsarten laufen, wobei nicht für jede Betriebsart alle Maschinenkomponenten eingesetzt werden. Bei der Festsetzung des Wartungsintervalls für eine bestimmte Komponente werden nur diejenigen Betriebsarten berücksichtigt, bei denen die Maschinenkomponente tatsächlich benutzt wird. So kann beispielsweise der Wartungshinweis bei einem Ventil lauten: "Ventildichtung wechseln", wobei das Kriterium für das Auftreten des Wartungshinweises von der Dauer des laufenden Wartungsintervalls dieses Ventils und von der Betriebsstundenzahl dieses Ventils abgeleitet ist.

Ferner kann das Wartungsintervall der Komponenten auf Basis der Verschleiß erzeugenden Zyklen der Komponenten beruhen. Dies sind bei Ventilen beispielsweise Schaltzyklen, also ein Öffnen und anschließendes Schließen des Ventils. Die Anzahl der erfolgten Zyklen kann ermittelt werden über die Steuerung des Ventils, die jede einzelne Ansteuerung zählt, oder über eine separate Messeinrichtung, die beispielsweise Druckstöße durch das Öffnen des Ventils ermittelt. Die Geschwindigkeit oder Frequenz, mit der das Ventil schaltet, ist dabei für den Verschleiß von untergeordneter Rolle. Jedoch unterscheiden sich die Anzahl der Schaltzyklen pro Zeit abhängig von Therapieart und Ventil stark. Bei Motoren besteht ein Verschleiß erzeugender Zyklus aus einer Motorumdrehung, die ebenfalls über das Steuersignal oder eine Messung am Motor ermittelt werden kann. Ebenfalls ein wichtiger Verschleißfaktor ist die Anzahl der durchgeführten thermischen und chemischen Belastungen während der Desinfektion der Komponenten. Jede durchgeführte Desinfektion kann z. B. bei Dichtelementen als Verschleiß erzeugender Zyklus bezeichnet und gezählt werden. Die Anzahl der Verschleiß erzeugenden Zyklen pro Zeit ist abhängig von der Betriebsart des Gerätes und der betrachteten Komponente sehr unterschiedlich.

Die Anzahl der Verschleiß erzeugenden Zyklen des laufenden Wartungsintervalls oder der tatsächlichen Betriebsstunden werden für die Komponenten separat ermittelt und gespeichert. Es besteht die Möglichkeit, für mehrere sicherheitsrelevante kritische Maschinenkomponenten die Wartungskriterien separat anzuzeigen. Dabei kann die Wartungsüberwachungseinrichtung auch so ausgebildet sein, dass sie bei Auftreten eines Wartungszeitpunktes für eine bestimmte Maschinenkomponente vorausberechnet, ob die Wartungsintervalle anderer Maschinenkomponenten ebenfalls innerhalb der nächsten Zeit ablaufen, so dass dann sämtliche demnächst anstehenden Wartungsvorgänge zusammengefasst werden können.

Generell ist es zweckmäßig, eine Anzeige für den erwarteten Zeitpunkt der nächsten Wartung vorzunehmen. Dabei kann beispielsweise eine Größe angezeigt werden, die die noch zu erwartende Restlaufzeit des laufenden Wartungsintervalls im Verhältnis zu der Länge des Wartungsintervalls angibt.

Im Folgenden wird unter Bezugnahme auf die Zeichnung ein Ausführungsbeispiel der Erfindung näher erläutert.

In der Zeichnung ist ein Zeitdiagramm der Wartungsintervallbestimmung der Dialysemaschine dargestellt.

Dem Diagramm ist entlang der Abszisse die Zeit t seit dem Zeitpunkt "O" der letzten Wartung in Monaten aufgetragen. Die Dialysemaschine enthält eine Einrichtung zur Zeitzählung des laufenden Wartungsintervalls. Die Dauer dieses Wartungsintervalls ist nicht fest vorgegeben, sondern wird nach den im Einzelnen zu erläuternden Kriterien bestimmt. Bei der Wartung stellt der Wartungstechniker das Wartungsintervall wieder auf 0, so dass ein neues Wartungsintervall beginnt.

Längs der Ordinate ist in der Zeichnung die Zahl der tatsächlichen Betriebsstunden OH (Operation Hours) des laufenden Wartungsintervalls aufgetragen. Auch diese Zahl wird zu Beginn des Wartungsintervalls auf 0 gestellt.

Die gestrichelte Linie 10 stellt die maximal mögliche Zahl der Betriebsstunden dar, die sich ergeben würde, wenn die Maschine neun Monate lang täglich 24 Stunden in Betrieb wäre. Die Untergrenze UGI des laufenden Wartungsintervalls ist im vorliegenden Beispiel mit neun Monaten angesetzt. Dies bedeutet, dass unabhängig von der Betriebsstundenzahl die Dauer des Wartungsintervalls mindestens neun Monate beträgt. Die Obergrenze OGH für die Betriebsstundenzahl beträgt bei dem dargestellten Ausführungsbeispiel 4.000 Betriebsstunden. Ferner ist eine Obergrenze OGI für die Dauer des laufenden Wartungsintervalls vorgesehen. Diese Obergrenze OGI liegt bei 18 Monaten. Selbst wenn die Maschine überhaupt nicht benutzt wurde, endet das Wartungsintervall nach 18 Monaten.

Einen möglichen Verlauf der Maschinenbenutzung gibt die Kurve 12 an, wobei die Steilheit der Kurve 12 die Häufigkeit der Benutzung repräsentiert. Der Punkt A stellt den aktuellen Status im gegenwärtigen Zeitpunkt $t_A$ dar. Die Recheneinrichtung berechnet die mittlere Steigung der Kurve 12 zwischen den Punkten O und A und ermittelt daraus die Gerade 14, deren Verlängerung 14a die Obergrenze OGH der Betriebsstundenzahl im Punkt E schneidet. Daraus ergibt sich, dass bei Fortsetzung des Betriebes mit der mittleren Intensität der Kurve 12 das erwartete Ende des Wartungsintervalls in Punkt E liegt, also bei etwa 16 Monaten.

Die Kurve 16 betrifft einen Fall sehr häufiger Benutzung. In diesem Fall wird die Obergrenze von 4.000 Stunden vor Ablauf der 9 Monate überschritten. Das Wartungsintervall würde dann an der Untergrenze UGI der Dauer des laufenden Wartungsintervalls enden.

Die Kurve 18 repräsentiert dagegen einen Fall von seltener Benutzung der Dialysemaschine. Das Wartungsintervall würde hier an der zeitlichen Obergrenze UGI des Wartungsintervalls von 18 Monaten enden. Die genannten Grenzen UGI, OGH, OGI können abhängig von den Marktanforderungen vor Ort unterschiedlich eingestellt werden. Hohe Haftungsrisiken machen unter Umständen engere Grenzen in Märkten oder bei bestimmten Kunden nötig.

Der abgelaufene Anteil X in Prozent der Gesamtlänge des Intervalls beträgt

$$X = \text{Länge AE/Gesamtlänge OE} * 100.$$

Dieser abgelaufene Anteil X wird in der Dialysemaschine laufend berechnet und angezeigt. Der Anteil X bildet eine Größe über die noch zu erwartende Restlaufzeit des laufenden Wartungsintervalls im Verhältnis zur Länge des Wartungsintervalls.

Andererseits besteht auch die Möglichkeit, das voraussichtliche Ende des Wartungsintervalls als Datum anzuzeigen.

Die Erfindung ist nicht auf die Anwendung der Wartungsanzeige bei einer Dialysemaschine beschränkt. Es besteht auch die Möglichkeit, das Prinzip der Wartungsanzeige bei Infusionspumpen anzuwenden, die ebenfalls einen hohen Sicherheitsstandard haben müssen.

**Patentansprüche**

1.    Dialysemaschine mit Wartungsanzeige, wobei die Dialysemaschine eine Messvorrichtung für die Messgrößen "Dauer des laufenden Wartungsintervalls" und "Betriebsstundenzahl innerhalb des laufenden Wartungsintervalls" und eine nach einem vorgegebenen Algorithmus arbeitende Recheneinrichtung enthält, mit der die Länge eines Wartungsintervalls bestimmt wird durch eine Kombination der Messgrößen: Dauer des laufenden Wartungsintervalls und abgelaufene Betriebsstundenzahl innerhalb des laufenden Wartungsintervalls.

2.    Dialysemaschine nach Anspruch 1, wobei das Ende des Wartungsintervalls bestimmt wird durch das Erreichen einer Obergrenze (OGH) für die Betriebsstundenzahl, jedoch nicht früher als eine Untergrenze (UGI) der Dauer des laufenden Wartungsintervalls.

3.    Dialysemaschine nach Anspruch 1 oder 2, wobei bei der Bestimmung der Länge eines Wartungsintervalls das Lebensalter der Dialysemaschine berücksichtigt wird.

4.    Dialysemaschine nach einem der Ansprüche 1 bis 3, wobei bei der Bestimmung der Länge eines Wartungsintervalls die Gesamtzahl der Betriebsstunden seit Herstellung der Maschine berücksichtigt wird.

5.    Dialysemaschine nach einem der Ansprüche 1 bis 4, wobei für einzelne Maschinenkomponenten, wie eine Pumpe oder ein Ventil, ein eigenes Komponenten-Wartungsintervall bestimmt wird und eine Komponenten-Wartungsanzeige erzeugt wird.

6.    Dialysemaschine nach Anspruch 5, wobei bei der Erzeugung der Wartungsanzeige für eine Maschinenkomponente nur diejenigen Betriebsarten der Maschine berücksichtigt werden, bei denen die Komponente benutzt wird.

7.    Dialysemaschine nach Anspruch 5 oder 6, wobei bei der Bestimmung der Länge eines Wartungsintervalls für eine Maschinenkomponente die Anzahl tatsächlich erbrachter verschleißrelevanter Zyklen wie Schaltspiele oder Rotationen der Maschinenkomponente berücksichtigt werden.

8.    Dialysemaschine nach einem der Ansprüche 5 bis 7, wobei bei der Bestimmung der Länge eines Wartungsintervalls für die Dialysemaschine die Komponenten-Wartungsintervalle berücksichtigt werden.

9.    Dialysemaschine nach Anspruch 5, wobei für mehrere Maschinenkomponenten die Betriebsstunden oder Zyklen separat erfasst werden.

10.  Dialysemaschine nach einem der Ansprüche 1 bis 9, wobei eine Anzeige für den erwarteten Zeitpunkt der nächsten Wartung anhand der Dauer des laufenden Wartungsintervalls und der darin enthaltenen Betriebsstundenzahl erfolgt.

11. Dialysemaschine nach einem der Ansprüche 1 bis 10, wobei eine Größe (X) angezeigt wird, die die noch zu erwartende Restlaufzeit des laufenden Wartungsintervalls im Verhältnis zur Länge des Wartungsintervalls angibt.

Claims

1. A dialysis machine with servicing indication, the dialysis machine including a measuring device for the parameters "duration of the present servicing interval" and "number of operating hours in the present servicing interval" and a calculating unit operating according to a predetermined algorithm, with which unit the duration of a servicing interval is determined by a combination of the parameters: duration of the present servicing interval and number of operating hours lapsed in the present servicing interval.

2. The dialysis machine of claim 1, wherein the end of the servicing interval is determined by reaching an upper limit (OGH) of the number of operating hours, but not before reaching a lower limit (UGI) of the duration of the present servicing interval.

3. The dialysis machine of claim 1 or 2, wherein the service life time of the dialysis machine is taken into account in determining the duration of a servicing interval.

4. The dialysis machine of one of claims 1 to 3, wherein the total number of operating hours since the manufacturing of the machine is taken into account in determining the duration of a servicing interval.

5. The dialysis machine of one of claims 1 to 4, wherein, for individual machine components such as a pump or a valve, a specific component servicing interval is determined and a component servicing indication is generated.

6. The dialysis machine of claim 5, wherein upon generating a servicing indication for a machine component only those modes of the machine are considered in which the component is used.

7. The dialysis machine of claim 5 or 6, wherein upon determining the duration of a servicing interval for a machine component, the number of actually performed wear-relevant cycles, such as switching cycles or rotations of the machine component, is taken into account.

8. The dialysis machine of one of claims 5 to 7, wherein the component servicing intervals are taken into account when determining the duration of a servicing interval for the dialysis machine.

9. The dialysis machine of claim 5, wherein the operating hours or cycles are determined separately for a plurality of machine components.

10. The dialysis machine of one of claims 1 to 9, wherein an indication of the expected time of the next servicing is made based on the duration of the present servicing interval and the number of operating hours included therein.

11. The dialysis machine of one of claims 1 to 10, wherein a value (X) is displayed that indicates the expected remaining duration of the present servicing interval relative to the duration of the service interval.

Revendications

1. Machine de dialyse avec affichage de la maintenance, comportant un dispositif de mesure des grandeurs "Durée d'un intervalle de maintenance en cours" et "Nombre d'heures de fonctionnement dans l'intervalle de maintenance en cours" et un dispositif de calcul fonctionnant selon un algorithme prédéfini et permettant de déterminer la longueur d'un intervalle de maintenance grâce à une combinaison des grandeurs mesurées : durée de l'intervalle de maintenance en cours et nombre d'heures de fonctionnement écoulées dans l'intervalle de maintenance en cours.

2. Machine de dialyse selon la revendication 1, dans laquelle la fin de l'intervalle de maintenance est déterminée par l'atteinte d'une limite supérieure (OHG) pour le nombre d'heures de fonctionnement, mais pas avant une limite inférieure (OGH) de la durée de l'intervalle de maintenance en cours.

**3.** Machine de dialyse selon la revendication 1 ou la revendication 2, dans laquelle on tient compte de son âge pour déterminer la longueur d'un intervalle de maintenance.

**4.** Machine de dialyse selon l'une quelconque des revendications 1 à 3, dans laquelle on tient compte du nombre total d'heures de fonctionnement de celle-ci depuis sa fabrication pour déterminer la longueur d'un intervalle de maintenance.

**5.** Machine de dialyse selon l'une quelconque des revendications 1 à 4, dans laquelle on détermine un intervalle de maintenance propre à différents composants de la machine comme une pompe ou une soupape, et on génère un affichage de la maintenance de ces composants.

**6.** Machine de dialyse selon la revendication 5, dans laquelle on ne tient compte que des modes de fonctionnement de celle-ci dans lesquels le composant est utilisé pour générer l'affichage de la maintenance de ce dernier.

**7.** Machine de dialyse selon la revendication 5 ou la revendication 6, dans laquelle on tient compte du nombre de cycles engendrant une usure réellement fournis tels que les cycles de travail ou les rotations de ses composants pour déterminer la longueur d'un intervalle de maintenance pour un composant.

**8.** Machine de dialyse selon l'une quelconque des revendications 5 à 7, dans laquelle on tient compte des intervalles de maintenance des composants pour déterminer la longueur d'un intervalle de maintenance de la machine.

**9.** Machine de dialyse selon la revendication 5, dans laquelle on saisit séparément les heures de fonctionnement ou cycles pour plusieurs de ses composants.

**10.** Machine de dialyse selon l'une quelconque des revendications 1 à 9, dans laquelle la date attendue de la prochaine maintenance est affichée à l'aide de la durée de l'intervalle de maintenance en cours et du nombre d'heures de fonctionnement qu'elle contient.

**11.** Machine de dialyse selon l'une quelconque des revendications 1 à 10, dans laquelle une grandeur (X) s'affiche qui indique le temps restant à attendre de l'intervalle de maintenance en cours par rapport à la longueur de l'intervalle de maintenance.

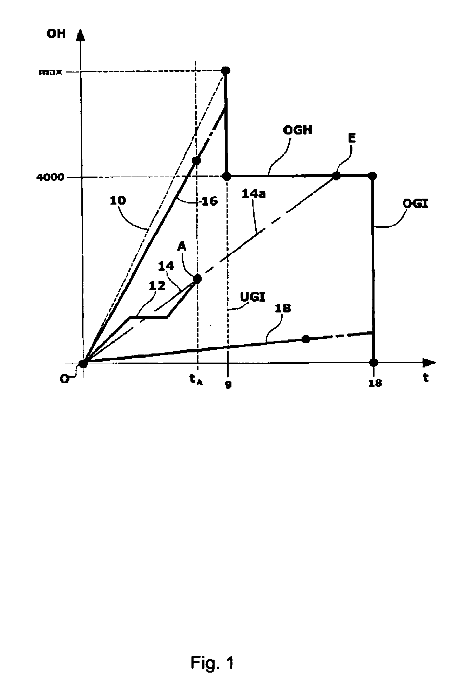

Fig. 1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1331589 A2
- WO 9640316 A

- DE 19832825 A1